# EUROPÄISCHE PATENTSCHRIFT

(11) **EP 1 420 253 B1**
(45) Veröffentlichungstag und Bekanntmachung des Hinweises auf die Patenterteilung: **31.05.2006**
(21) Anmeldenummer: 02090300.1
(22) Anmeldetag: 23.08.2002
(51) Int. Cl.: G01N 33/569

(54) **Verfahren zum Nachweis und zur Isolierung von T-Lymphozyten, die ein definiertes Antigen erkennen**
Method of detecting and isolating T-lymphocytes that recognize a defined antigen
Procédé de détection et isolation des lymphocytes T reconnaissant un antigène defini

(43) Veröffentlichungstag der Anmeldung: 19.05.2004
(73) Patentinhaber: Deutsches Rheuma-Forschungszentrum Berlin, 10117 Berlin (DE)
(72) Erfinder: Frentsch, Marco, Dipl.-Bioch., 10785 Berlin (DE); Rothe, Martin, 13055 Berlin (DE); Thiel, Andreas, Dr., 10961 Berlin (DE)
(74) Vertreter: Ziebig, Marlene

(56) Entgegenhaltungen:
- BERNER B ET AL: "INCREASED EXPRESSION OF CD40 LIGAND (CD154) ON CD4+ T CELLS AS A MARKER OF DISEASE ACTIVITY IN RHEUMATOID ARTHRITIS" ANNALS OF THE RHEUMATIC DISEASES, BRITISH MEDICAL ASSOCIATION, LONDON, GB, Bd. 59, 2000, Seiten 190-195, XP002937171 ISSN: 0003-4967
- SCHOENBECK U ET AL: "CD154 (CD40 LIGAND)" INTERNATIONAL JOURNAL OF BIOCHEMISTRY AND CELL BIOLOGY, EXETER, GB, Bd. 32, Nr. 7, Juli 2000 (2000-07), Seiten 687-693, XP001053568 ISSN: 1357-2725
- FISHER P J ET AL: "Immunomagnetic separation reagents as markers in electron microscopy" JOURNAL OF IMMUNOLOGICAL METHODS, ELSEVIER, AMSTERDAM, NL, Bd. 262, Nr. 1-2, 1. April 2002 (2002-04-01), Seiten 95-101, XP004352178 ISSN: 0022-1759
- DATABASE BIOSIS [Online] BIOSCIENCES INFORMATION SERVICE, PHILADELPHIA, PA, US; 20. März 2002 (2002-03-20) LEE BYUNG O ET AL: "The biological outcome of CD40 signaling in B lymphocytes is dependent on the duration of CD154 expression on T cells: Reciprocal regulation by IL-4 and IL-12." Database accession no. PREV200200353975 XP002228655 & FASEB JOURNAL, Bd. 16, Nr. 4, 20. März 2002 (2002-03-20), Seite A350 Annual Meeting of the Professional Research Scientists on Experimental Biology;New Orleans, Louisiana, USA; April 20-24, 2002, March 20, 2002 ISSN: 0892-6638

## Beschreibung

Die Erfindung betrifft die Verwendung von CD154 zum in vitro Nachweis und zur Isolierung von T-Lymphozyten, die ein definiertes Antigen erkennen, und ein Verfahren zum Nachweis und zur Isolierung von T-Lymphozyten in Zellsuspensionen oder Körperflüssigkeiten, die ein definiertes Antigen erkennen.

Das Immunsystem besteht aus Zellen und Molekülen, die im Körper zirkulieren und deren Homeostase und Kooperation durch Zytokine und Wachstumsfaktoren aufeinander abgestimmt werden. Nicht benötigte Zellen werden durch Apoptose eliminiert. Diese Mechanismen werden durch äußere Einflüsse oder genetische Prädispositionen verschiedenster Art beeinflusst. Das Immunsystem spielt eine zentrale Rolle bei der Entstehung vieler Erkrankungen, insbesondere Allergien, Entzündungen und Autoimmunerkrankungen. Es ist verantwortlich für eine verstärkte Immunität nach Impfungen, versagt jedoch bei der Entstehung von Tumorerkrankungen.

Dendritische Zellen (DC) sind die Wächter des Immunsystems und im Körper dort lokalisiert, wo fremde und möglicherweise gefährliche Stoffe eindringen können. Sie sind professionelle Antigen-präsentierende Zellen, die das Antigen aufnehmen, verdauen, Peptidfragmente des Antigens spezifischen T-Lymphozyten präsentieren und sie dabei aktivieren. Die unreifen Vorläufer der DC wandern kontinuierlich über das Blut in die Lymphorgane, wo sie unter bestimmen Umständen zu reifen DC differenzieren können. Es gibt verschiedene Vorläuferzellen, die zu unterschiedlichen Typen von DC ausreifen können. Diese unterschiedlichen Typen von DC reagieren auch unterschiedlich auf Antigene durch die Expression bestimmter Membranmoleküle und Zytokine. Auf diese Weise kontrollieren sie die Aktivierung der T-Lymphozyten, die dann pro- (Th1) oder antiinflammatorisch (Th2), regulatorisch (Tr) oder tolerant (Ta) werden können. Die Art der Aktivierung der T-Lymphozyten wird durch kostimulatorische Aktivierungssignale von Antigenpräsentierenden Zellen bestimmt. Aktivierungssignale sind Liganden für Rezeptoren der T-Lymphozyten. Diese Liganden befinden sich auf der Oberfläche der APC, sie sind an die extrazelluläre Matrix gebunden oder sie werden von Zellen sezerniert, so die Zytokine. Neben der Antigen-spezifischen Aktivierung durch Signale über den Antigenrezeptor der T-Lymphozyten und kostimulierende Liganden ist jedoch auch eine unspezifische Aktivierung von T-Lymphozyten beschrieben; z. B. durch Zytokine oder durch Lektine.

Die Gesamtheit der molekularen Veränderungen bei der Aktivierungsreaktion und funktionellen Differenzierung von T-Lymphozyten ist jedoch derzeit nur unvollständig aufgeklärt. So ist auch die Erfassung des Aktivierungs- und Funktionzustandes von T-Lymphozyten, die ein bestimmtes Antigen erkennen, beim gegenwärtigen Stand der Technik nur ungenau möglich.

Für klinische Anwendungen bei der Dignostik von Krankheiten und in der Forschung ist es essentiell, insbesondere solche spezifischen T-Lymphozyten erfassen zu können, die durch ein bestimmtes Antigen aktiviert werden können oder aktivierbar sind. Weiterhin ist es wünschenswert, solche Antigen-spezifischen T-Lymphozyten aus Zellgemischen insbesondere für zelltherapeutische Zwecke isolieren zu können.

Im Stand der Technik sind mehrere Verfahren offenbart, mit denen der Aktivierungs- und Funktionszustand von T-Lymphozyten bestimmt werden kann. So offenbart die DE 100 21 834 A1 mRNA-Moleküle zur Verwendung als Indikatoren für den Funktionszustand von T-Lymphozyten, wobei die mRNA-Moleküle bei aktivierten T-Lymphozyten im Vergleich zum Normal- bzw. zum Ruhezustand vermehrt oder vermindert exprimiert sind. Durch Bestimmung der Interaktion zwischen den komplementären, hybridisierten Nucleinsäuresequenzen ist es möglich, Unterschiede in der Expressionsstärke der mRNA-Moleküle zu bestimmen, was Rückschlüsse auf den Aktivierungszustand der T-Lymphozyten zulässt. Weiterhin offenbart diese Druckschrift die Verwendung von Polypeptiden, die durch die Nucleotidsequenzen codiert werden und gegen die Polypeptide gerichtete Antikörper sowie die Verwendung dieser Antikörper zum Nachweis des Aktivierungs- und Funktionszustandes von T-Lymphozyten.

Die DE 37 37 703 A1 offenbart Antikörper, die gegen ein neutrophil aktivierendes Polypeptid gerichtet sind und als Diagnostikum eingesetzt werden können, um aktivierte Strukturen der zellulären Immunantwort zu detektieren und zu charakterisieren.

Die DE 42 26 974 C2 offenbart ein Verfahren und eine Vorrichtung zur Aufbereitung und Separation von Zellsuspensionen. Die beschriebene Separationsvorrichtung und das Verfahren zur kontinuierlichen Aufbereitung einer Zellsuspension kann verwendet werden, um unterschiedliche Bestandteile, beispielsweise des menschlichen Blutes zu separieren, wobei Zellen, die im Zusammenhang mit der zellulären Immunantwort stehen, spezifisch aufgereinigt werden können.

In der US-PS 5,874,302 wird ein Verfahren offenbart, mit dem T-Lymphozyten kultiviert werden und die so gewonnenen T-Lymphozyten zu klinischen Behandlungen von Tumoren eingesetzt werden. Gemäß der US-PS 5,874,302 wird Blut als Ausgangsmaterial für die Herstellung der T-Zellkultur-Suspension verwendet, wobei das entsprechende Startmaterial mit bestimmten Tumorzellen cokultiviert wird.

Die CA 1,296,622 offenbart ein Verfahren und eine Vorrichtung zum Bestimmen des immunregulatorischen Status von Leukozyten. Die Leukozyten werden mit Standardsubstanzen stimuliert, um den immunregulatorischen Status, beispielsweise mit Hilfe von markierten Antikörpern zu bestimmen. Zunächst wird hierzu eine Probe aus peripheren mononukleären Zellen des Patienten, der ein Immun-beeinflussendes Mittel erhalten hat, vermehrt und anschließend wird in einem Aliquot der vermehrten Probe der Prozentsatz von mononukleären Zellen bestimmt, welche ein Aktivierungs-Antigen in vitro exprimieren. Anschließend wird der Prozentsatz mit einem vorgegebenen Wert des Prozentsatzes als Anzeige des in vitro Effektes verglichen, wodurch der immunregulatorische Zustand des Patienten bzw. des Effektes der Immun-beeinflussenden Therapie auf den immunregulatorischen Zustand bestimmt werden kann. Zu den Unterklassen der zu bestimmenden mononukleären Zellen gehören beispielsweise T-Lymphozyten, cytotoxische T-Lymphozyten und Helfer-T-Lymphozyten.

In der WO 99/58977 wird ein Verfahren zur direkten Selektion von Antigen-spezifischen T-Lymphozyten beschrieben. Die T-Lymphozyten werden hier anhand der Produktion von Zytokinen mittels Durchfluss-Zytometrie oder der magnetischen Zellsortierung separiert. Es werden hier solche aktivierte T-Lymphozyten selektiert, die nach Stimulation mit Antigen ein bestimmtes oder mehrere bestimmte Zytokine exprimieren.

In Berner et al, Annals of the Rheumatic Diseases, Bd. 59, 2000, S. 190-195, wird ein Verfahren zum Nachweis von Antigen-aktivierten T-Zellen mittels Bestimmung der CD154 Expression bei Patienten, die an rheumatoider Arthritis leiden, beschrieben. Die erhöhte Expression von CD154 in CD4 T-Lymphozyten wird mit erhöhter CD4 T-Zell-Aktivierung und erhöhter Krankheitsaktivität in Verbindung gebracht.

Weiterhin ist es mit den bekannten diagnostischen Verfahren nicht möglich, die Aktivierung durch eine virale oder bakterielle Infektion von einer Aktivierung durch eine Autoimmunreaktion oder durch eine Transplantatabstoßung zu unterscheiden, obwohl eine solche Unterscheidung aus diagnostischen und aus klinischen Gesichtspunkten heraus wünschenswert ist. Durch die unzureichende Detektion von aktivierten T-Lymphozyten in einer biologischen Probe ist es auch nicht möglich, diese entsprechenden Zellen gezielt zu separieren, da sie hierfür zunächst eindeutig bestimmt werden müssen.

Bisher sind keine immundiagnostischen Verfahren beschrieben, die einen schnellen und einfachen Nachweis der Gesamtheit aller für ein Antigen spezifischen T-Lymphozyten und darüber hinaus deren Isolierung ermöglichen. Bei den verschiedensten Erkrankungen ist es von größtem diagnostischem und therapeutischen Interesse, die die Immunantworten zentral und somit auch verschiedenste Immunpathogenesen steuernden T-Lymphozyten direkt Antigenspezifisch nachzuweisen oder sie für zelltherapeutische Ansätze, wie den adoptiven T-Zelltransfer, zu isolieren. Dies betrifft Erkrankungen wie Allergien und Autoimmunerkrankungen, denen fehlgesteuerte Immunreaktionen zu Grunde liegen wie auch chronische Infektionskrankheiten, Tumore oder Leukämien.

Bisherigen Verfahren liegen zwei unterschiedliche Strategien zugrunde:
(i) Der "strukturelle" Nachweis Antigen-spezifischer T-Lymphozyten basiert auf der direkten Markierung der von den T-Lymphozyten ausgeprägten T-Zellrezeptoren mittels MHC-Multimeren, die zusammen mit den jeweiligen antigenen Peptiden komplexiert sind (US 5,635,363 / FR 9911133). Die Nachteile solcher Verfahren bestehen darin, dass sie zum einen abhängig von dem der zu untersuchenden Individuen ausgeprägten MHC-Genotyp sind und zum anderen dass die MHC-Moleküle nur mit einem immundominanten Peptidepitop des jeweiligen Antigens komplexiert werden. Jedes Agens muss somit individuell entsprechend des Genotyps und zu verwendenden immundominanten Peptidepitops, das zudem nur bei wenigen Antigenen und auch nur für wenige MHC-Allele bekannt ist, des Antigens hergestellt werden. Zudem konnten bisher die für den Nachweis und die Isolierung MHC-II restringierter Antigen-spezifischer CD4+ Th-Lymphozyten essentiellen MHC-II Multimere aufgrund besonderer struktureller Merkmale des MHC-II Moleküls nicht in für eine Anwendung ausreichender Qualität hergestellt werden.
(ii) Funktionale Nachweise Antigen-spezifischer T-Lymphozyten beruhen entweder auf der Antigen-reaktiven Proliferation nach Stimulation mit Antigen, die jedoch erst nach 3-5 Tagen detektierbar ist, oder auf Antigen-reaktiver Expression von Aktivierungsmolekülen oder Sekretion von Zytokinen. Während bisher beschriebene Aktivierungsmarker zum Teil auch von speziellen nicht aktivierten T-Lymphozyten ausgeprägt werden und daher nicht spezifisch genug sind, kann anhand der "reaktiven" Expression von Zytokinen nicht die Gesamtheit der spezifischen T-Lymphozyten nachgewiesen werden. Durch die unzureichende Detektion von spezifisch aktivierten T-Lymphozyten in einer biologischen Probe ist somit auch nicht möglich, diese entsprechenden Zellen gezielt zu separieren, da sie hierfür zunächst eindeutig bestimmt werden müssten.

Die bekannten Verfahren, bei denen spezifische T-Lymphozyten aufgrund ihrer Zytokin Sekretion detektiert und isoliert werden, beispielsweise nach der WO 99/58977, kann die Gesamtheit aller spezifischen T-Lymphozyten nicht bestimmt und isoliert werden, da sich oft jede aktivierte T-Zelle durch individuelle Zytokinprogramme auszeichnet.

Nachteilig bei den bekannten Verfahren der Isolierung von lebenden T-Lymphozyten anhand der Produktion von Zytokinen ist weiterhin, dass die Zellen zum Zeitpunkt ihrer maximalen Sekretionsleistung aus der Kultur herausgenommen werden, um an ihnen eine für die jeweiligen Zytokine spezifische "Fangmatrix" anzbringen. Danach müssen die Zellen für eine kurze Zeit wieder kultiviert werden. Dies erhöht den technischen Aufwand zur Gewinnung spezifischer T-Lymphozyten beträchtlich. Gerade in den letzten Jahren wurden in verschiedenen Veröffentlichungen immer wieder regulatorische T-Lymphozyten (Tr/Treg) beschrieben, denen sich keine definierten Zytokinmuster zuordnen lassen, d.h. die Zellen sind anhand einer Zytokinsekretion nicht detektierbar oder isolierbar. Zudem ist die Expression bestimmter Zytokine (z. B. IL-10) auch entscheidend von der Art der gewählten in vitro Aktivierung abhängig.

Der bisherige Nachweis und die Isolierung von spezifischen T-Lymphozyten ist bisher auch deshalb limitiert, da zwar eine Reihe von Biomolekülen bekannt sind, die als Indikatoren bzw. Marker für bestimmte aktivierte T-Lymphozyten herangezogen werden können, jedoch ist es derzeit aufgrund der begrenzten Zahl und Qualität der verfügbaren Aktivierungsmarker nicht möglich, durch Bestimmung eines dieser Marker zu entscheiden, ob die T-Lymphozyten sich in dem gewünschten Zustand befinden. Beispielsweise wird der Aktivierungsmarker CD69 von allen T-Lymphozyten nach Aktivierung exprimiert. Jedoch führen auch jegliche Stresseinwirkungen zur Expression von CD69, so dass durch Bestimmung dieses Markers keine Aussage über die Antigen-spezifischen T-Lymphozyten möglich ist.

Aufgabe der vorliegenden Erfindung ist es daher Verfahren bereitzustellen, mit denen alle Antigen-spezifischen T-Lymphozyten unabhängig von ihrem funktionalen Potential einfach, kostengünstig und sicher detektiert bzw. isoliert werden können.

Die vorliegende Erfindung löst dieses technische Problem durch eine Verwendung von CD154 zum Nachweis und/oder zur Isolierung von T-Lymphozyten, die ein bestimmtes Antigen erkennen.

CD154 ist ein Mitglied der TNF-Gen-Familie und wird unter anderem von verschiedenen Zellen, insbesondere T-Lymphozyten exprimiert. CD154 wird ein bis drei Stunden nach der Aktivierung von den stimulierten T-Lymphozyten herunterreguliert.

Lymphozyten stellen die spezifischen Träger der Immunantwort dar, wobei zwei große Populationen bekannt sind: Die B- und die T-Lymphozyten. Die T-Lymphozyten besitzen zahlreiche Oberflächenmoleküle, von denen das CD4- und das CD8-Protein von besonderer Bedeutung sind. Die CD4 T-Lymphozyten werden auch als Helfer-T-Zellen (Th-Zellen) bezeichnet, da sie über die Produktion löslicher Botenstoffe bei der Aktivierung anderer Zellen mithelfen.

Lymphozyten und andere Leukozyten exprimieren viele verschiedene Moleküle auf ihrer Zelloberfläche. Einige erscheinen nur kurzzeitig während bestimmter Phasen der Differenzierung oder nach Aktivierung des Zellen. Andere Moleküle sind konstant und typisch für die jeweilige Zellreihe. Solche konstant exprimierenden Antigene können als Marker für bestimmte Zellpopulationen genutzt werden. Es gibt eine einheitliche Nomenklatur für Zelloberflächenmarker, das CD-System (CD = cluster of differentiation), in welchem die Marker fortlaufend nummeriert sind. So kommen beispielsweise auf NK- und T-Lymphozyten CD 154 wie auch CD 165 oder andere Moleküle vor.

Überraschend war, dass CD154 verwendet werden kann, um T-Lymphozyten unabhängig von ihrem funktionalen Potential nachzuweisen. Der überraschende Vorteil der Verwendung von CD154 zum Nachweis und zur Separation von T-Lymphozyten liegt darin, dass die T-Lymphozyten unabhängig von ihrer Funktion sicher detektiert bzw. isoliert werden können, d.h. alle antigenspezifischen T-Lymphozyten in einer Probe können bestimmt und separiert werden. Somit wird durch die erfindungsgemäße Verwendung vorteilhafterweise ein neuer praktikabler Kandidat für die Bestimmung von antigenspezifischen T-Lymphozyten bereitgestellt.

Nach einer besonderen Ausführungsform der Erfindung sind die T-Lymphozyten Th-Lymphozyten, insbesondere CD4⁺ und/oder CD8⁺ Th-Lymphozyten. Für die Antigenerkennung benutzen T-Zell-Lymphozytenpopulationen eine T-Zellrezeptor (TZR), der ein heterodimeres Molekül darstellt, das aus verschiedenen Kettenkombinationen besteht. Der weitaus größte Teil aller T-Lymphozyten trägt einen alpha-/beta-T-Zellrezeptor. Diese T-Lymphozyten besitzen weitere Oberflächenmoleküle, insbesondere das CD4- und das CD8-Protein. CD8-Lymphozyten werden von Antigenen stimuliert, die von Klasse-I-Molekülen des MHC präsentiert werden und sind als zytolytische T-Lymphozyten für die Virusabwehr von entscheidender Bedeutung. CD4-T-Lymphozyten erkennen Antigene, welche von MHC-Klasse-II-Molekülen präsentiert werden und haben einen wesentlichen Anteil an der Abwehr von Bakterien, Pilzen, Protozonen und anderen Parasiten.

In einer weiteren bevorzugten Ausführungsform der Erfindung werden inflamatorische, antiinflamatorische, regulatorische und/oder suppressive T-Lymphozyten nachgewiesen und/oder gewonnen.

Die Erfindung betrifft auch ein Verfahren zum Nachweis und/oder Isolierung von antigenspezifischen T-Lymphozyten in einer Suspension nach Aktivierung mit einem Antigen, wobei die Suspension mit einem CD40/CD154-System-Inibotor in Kontakt gebracht wird, CD154 intra- oder und/oder extrazellulär bestimmt und die CD154 aufweisenden Zellen nachgewiesen und/oder isoliert werden.

Es ist überraschend, dass durch das "Inkontakt" bringen der zu untersuchenden Suspension oder Probe mit einem CD40/CD154 System-Inhibitor CD154 intra- und/oder extrazellulär bestimmbar und somit die Zellen, die CD154 aufweisen, nachgewiesen und isoliert oder separiert werden können, wobei es sich bei diesen Zellen insbesondere um die Gesamtheit der Antigen-spezifischen CD4⁺ Th-Lymphozyten handelt.

CD40 ist ein 45-50 kDa großes membranständiges Glycoprotein aus der TNF-Rezeptor-Genfamilie (Tumor-Nekrose-Faktor-Rezeptor-Genfamilie). Es wird von verschiedenen hämatopoetischen Zelltypen aber auch von Eptithel- und Endothelzellen, Karzinomen, Fibroblasten und Muskelzellen ausgeprägt. Es kann von CD154 gebunden werden, das dementsprechend Mitglied der TNF-Gen-Familie ist und ebenfalls von vielen Zellen mit sehr unterschiedlichen Funktionen exprimiert werden kann. CD40 wird auf verschiedenen Zellen exprimiert, wie zum Beispiel B-Lymphozyten, dendritische Zellen, Monozyten/Makrophagen, Mastzellen, hämatopoetische Stamm-/Vorläuferzellen (human), Thymusepithelzellen (Maus), Endothelzellen, Fibroblasten (Maus), Mukelzellen (human) und/oder Karzinome (human). CD154 wird beispielsweise auf T-Lymphozyten, aktivierte dendritischen Zellen (human), Monozyten, Mastzellen (human), basophilen/eosinophilen Granulozyten (human), NK-Lymphozyten (Maus), fetale Thymozyten (Maus) und/oder B-Zellen (human) exprimiert.

Entsprechend der sehr heterogenen Expressionsmuster auf verschiedenen Zelltypen sind zahlreiche CD40/CD154 Wechselwirkungen beschrieben. Das CD40/CD154 System ist ein Beispiel für verschiedene Systeme, die auf Rezeptor-Liganden-Interaktionen basieren und als solche eine große Bedeutung bei Reaktionen des Immunsystems haben. Die CD40/CD154-Interaktionen zwischen T-Lymphozyten und dendritischen Zellen (DC) sind u.a. von Bedeutung bei der Induktion und Regulation der Immunantwort von Bedeutung. Eine spezielle Stellung nehmen CD40/CD154 Interaktionen bei Entwicklung von humoralen Antikörperreaktionen ein. Eine besondere klinische Bedeutung zeigte sich bei dem sogenannten X-chromosomalen Hyper-IgM-Syndrom; einer Immunerkrankung, bei der die Expression eines nichtfunktionellen CD154 zu einer stark verminderten Antikörperbildung und pyogenen Infektionen führt. In anderen Zellen beeinflusst die Signalweiterleitung über CD40/CD154 aber auch beispielsweise die Entstehung von Thromben in vivo und verstärkt die Entwicklung von Ateriosklerose.

Die Bedeutung des von T-Lymphozyten ausgeprägten CD154 ist somit vor allem während der Interaktion mit B-Lymphozyten für die Entwicklung von humoralen Antikörperantworten essentiell. Anscheinend kann das von T-Lymphozyten ausgeprägte CD154 auch mit von verschiedenen Antigenpräsentierenden Zellen (APC) exprimiertem CD40 in Interaktion treten. Dies wird deutlich, da während der in vitro Stimulation mit spezifischen Antigenen CD154 auf aktivierte T-Lymphozyten sehr schnell herunter reguliert wird. Als für dieses Phänomen verantwortlich angesehen wird die Interaktion von CD154 auf den aktivierten T-Lymphozyten mit von in solchen Systemen auch von APC wie Monozyten stark exprimierten CD40. Die spezifisch von aktivierten T-Lymphozyten ausgeprägten CD154 Moleküle werden danach in den Zellen degradiert. Es steht dann in einem nicht durch das erfindungsgemäße Verfahren beeinflussten System als Marker für spezifische CD4+ Th-Lymphozyten nicht mehr zur Verfügung.

Durch die Zugabe eines CD40/CD154 System-Inhibitors wird die Wechsel- und Signalwirkung sowie Interaktion von CD40 und CD154 gestört bzw. inhibiert. CD40/CD154 System-Inhibitoren können im Sinne der Erfindung alle Moleküle oder auch physikalische Einwirkungen sein, die in der Lage sind, die Interaktion zwischen CD40 und CD154 zu blockieren oder zu inhibieren. Demgemäß kann das inhibierende Mittel ein Antikörper, der beispielsweise gegen CD40 gerichtet ist, sein, ein Molekül, ein Cäsium- oder ein Lithiumion, welches die Wechselwirkung zwischen CD40 und CD154 untereinander beeinflusst. Es kann selbstverständlich aber auch eine Substanz sein, die die Sekretion oder Endozytose in der Zelle inhibiert, wie z.B. Brefeldin-A (Bref-A). Bref-A ist ein Inhibitor des Goldi-Apparates und der Sekretion einer Vielzahl an Zytokinen. Durch diese Stoffe wird gewährleistet, dass entweder CD40, CD154, die Wechselwirkung zwischen den beiden bzw. das CD40/CD154 System so modifiziert wird, dass CD154 entweder auf der Zelloberfläche nicht mehr herunter reguliert bzw. abgebaut wird, oder, sofern es sich noch innerhalb der Zelle befindet, nicht mehr innerhalb dieser transportiert wird. Durch die Unterbrechung des Transportes innerhalb der Zelle wird der Abbau von CD154 verhindert. Somit wird CD154 innerhalb oder außerhalb der Zelle als außenstehender Rezeptor stabilisiert und kann mit dem Fachmann bekannten Nachweisverfahren detektiert und folgend isoliert werden. Dem Fachmann sind verschiedene Verfahren und Vorrichtungen bekannt, mit denen detektierte Zellen des Immunsystems separiert, aufgereinigt, angereichert bzw. isoliert werden können, z.B. die Durchflusszytometrie oder die magnetische Zellsortierung.

Durch die Zugabe des CD40/CD154 System-Inhibitors wird der Abbau oder die Degradation von CD154 innerhalb und/oder außerhalb der Zelle unterbunden, so dass die Zellen, die CD154 aufweisen, charakterisiert und folgend anhand dieser Charakterisierung isoliert werden können. Durch das erfindungsgemäße Verfahren wird somit insbesondereder neue erfindungsgemäße Kandidat, CD154, für die Bestimmung von Antigen-spezifischen T-Lymphozyten bereitgestellt.

In einer bevorzugten Ausführungsform der Erfindung werden als T-Lymphozyten Th-Lymphozyten detektiert und/oder separiert, insbesondere CD4⁺ und/oder CD8⁺ Th-Lymphozyten.

In einer bevorzugten Ausführungsform der Erfindung ist der CD40/CD154 System-Inhibitor ein gegen CD40 gerichteter Antikörper, ein gegen CD154 gerichteter Antikörper, ein Sekretionsinhibitor und/oder ein Endozytoseinhibitor. Dem Fachmann sind verschiedene Sekretionsinhibitoren und Edozytoseinhibitoren bekannt. Der Antikörper kann hierbei ein polyklonaler, monoklonaler bzw. ein spezifisch modifizierter Antikörper oder ein Antikörperfragment, beispielsweise ein Antikörper sein, der mit einem Phagen oder einem Phagenfragment assoziiert ist. Er kann sowohl gegen ein einzelnes Epitop auf CD40 bzw. gegen mehrere Strukturen von CD40 gerichtet sein. Ein Antikörper im Sinne der Erfindung ist ein Molekül, welches CD40 so beeinflusst, dass eine Wechselwirkung mit CD154 vorteilhafterweise nicht mehr möglich ist. Antikörper bedeutet daher im Zusammenhang mit der Erfindung ein Polypeptid, das im Wesentlichen von Immunoglobulin-Genen oder Fragmenten davon codiert wird, das CD40 spezifisch bindet bzw. erkennt. Der in der vorliegenden Erfindung verwendete Begriff Epitop bedeutet eine beliebige Antigen-Determinante auf einem Antigen, an das das Paratop eines Antikörpers bindet. Epitop-Determinaten bestehen insbesondere aus chemisch aktiven Oberflächengruppierungen von Molekülen, wie Aminosäuren oder Zuckerseitenketten und besitzen normalerweise sowohl spezifische Merkmale der dreidimensionalen Struktur als auch spezifische Ladungsmerkmale.

In einer bevorzugten Ausführungsform der Erfindung werden als der Sekretionsinhibitor und/oder der Endozytosehibotor Brefeldin-A und/oder Monsensin verwendet. Brefeldin A ist ein Metabolit des Pilzes Penicillium brefeldianum und blockiert als carboxyliertes Ionophor den Transport neu synthetisierter Proteine vom endoplasmatischen Retikulum in den Golgi-Apparat und behindert den Austausch zwischen Endosomen und Lysomen während der Kreislauf zwischen Zellmembran und Endosomen vorteilhafterweise ungestört bleibt.

In einer weiteren, besonders bevorzugten Ausführungsform der Erfindung wird CD154 intrazellulär in fixierten Zellen detektiert. Da CD154 extrazellulär schnell herunterreguliert bzw. abgebaut wird, sobald eine antigen-spezifische Aktivierung von insbesondere Th-Lymphozyten stattgefunden hat, kann CD154 mit Vorteil intrazellulär mit hoher Genauigkeit in-vitro detektiert werden. Insbesondere durch die Zugabe von Brefeldin A wird der Transport des Proteins CD154 an die Zelloberfläche blockiert. Vorteilhafterweise ist hierdurch die Analyse des gesamten CD154, welches sich intrazellulär befindet, auf sehr spezifische Weise möglich, was eine nahezu vollständige Detektion von insbesondere allen reaktiven CD4⁺ Th-Lymphozyten ermöglicht. Durch die intrazelluläre Bestimmung von CD154 wird ein sehr gutes Signal-Rausch-Verhältnis erreicht, was eine sehr sichere und effiziente Bestimmung der Antigen-spezifischen Th-Lymphozyten ermöglicht.

In einer weiteren bevorzugten Ausführungsform der Erfindung wird CD154 extrazellulär auf vitalen Zellen detektiert. Um insbesondere Antigen-spezifische CD4⁺ und/oder CD8⁺ Th-Lymphozyten im therapeutischen oder klinischen Bereich einzusetzen, ist es erforderlich, die Zellen so zu detektieren, dass sie in ihrer Funktionalität nicht negativ beeinflusst werden, d.h., dass ein Nachweis vorteilhafterweise so gestaltet ist, dass die Zellen lebend und vital detektiert werden können, um sie folgend zur Herstellung eines Arzneimittels verwenden zu können. Es ist beispielsweise möglich, mittels anti-CD40-Antikörper CD40 zu neutralisieren, um den Abbau von CD154 auf der Zelloberfläche zu verhindern, was eine Detektion und Isolierung von Antigen-spezifischen CD4⁺ und/oder CD8⁺ Th-Lymphozyten erlaubt.

Es ist bevorzugt, die Antigen-spezifischen T-Lymphozyten, insbesondere CD4⁺ und/oder CD8⁺ aus Frischblut, gefrorenen Zellen, periphere mononukleäre Blutzellen (PBMC) und/oder aus anderen Körperflüssigkeiten zu gewinnen.

In einer bevorzugten Ausführungsform der Erfindung sind die isolierten und/oder separierten T-Lymphozytenzellen, die keine Cytokine produzieren bzw. die kein definiertes Zytokinmuster aufweisen. Mit Vorteil ist es also möglich, alle antigenspezifischen T-Lymphozyten, d. h. die Gesamtheit dieser Zellpopulation, die gegen ein bestimmtes Antigen gerichtet ist, zu detektieren und/oder zu isolieren, und zwar unabhängig davon, ob diese T-Lymphozyten Cytokine produzieren oder nicht oder ob die T-Lymphozyten sich keinen definiertem Zytokinmuster zuordnen lassen.

Das erfindungsgemäße Verfahren zur Detektion und zur Isolierung von Antigen-spezifischen T-Lymphozyten, beispielsweise CD4⁺ Th-Lymphozyten, erlaubt es, insbesondere Antigen-spezifische Th-Lymphozyten für die Zelltherapie gegen verschiedene Krankheiten, wie beispielsweise Krebs oder virale Infektionen einzusetzen. Bisher wurden z.B. Th-Lymphozyten in Form von Zelllinien oder Zellklonen für die adoptive Th-Lymphozytentherapie verwandt. Die Herstellung dieser Zelllinien bzw. Zellklonen ist jedoch sehr aufwendig und benötigt einen langen Zeitraum. Das erfindungsgemäße Verfahren erlaubt es erstmalig, Antigen-spezifische Th-Lymphozyten mittels der Detektion von CD154 in kurzer Zeit in vivo wie in vitro zu detektieren und zu isolieren. Somit ist es erstmals möglich, Th-Lymphozyten aufgrund ihrer Antigenreaktivität unabhängig von ihren Phänotyp zu selektieren.

Mit Vorteil ist es durch die Verwendung von CD154 als Marker für antigenspezifische T-Lymphozyten möglich, beispielsweise aus Körperflüssigkeiten oder künstlichen Zellsuspensionen gegen ein gewähltes Antigen oder gewählte Antigene spezifische T-Lymphozyten, insbesondere für zelltherapeutische Anwendungen, zu gewinnen. Vorteilhafterweise ist es möglich, spezifische T-Lymphozyten zur Expansion oder mit nachfolgendem oder direktem adoptiven Transfer zu isolieren, wobei diese spezifischen T-Lymphozyten inflamatorische oder antiinflamatorische und insbesondere regulatorische oder supressive T-Lymphozyten darstellen.

Im folgenden soll die Erfindung anhand von Beispielen veranschaulicht werden, ohne die Erfindung darauf zu beschränken.

### Beispiel 1

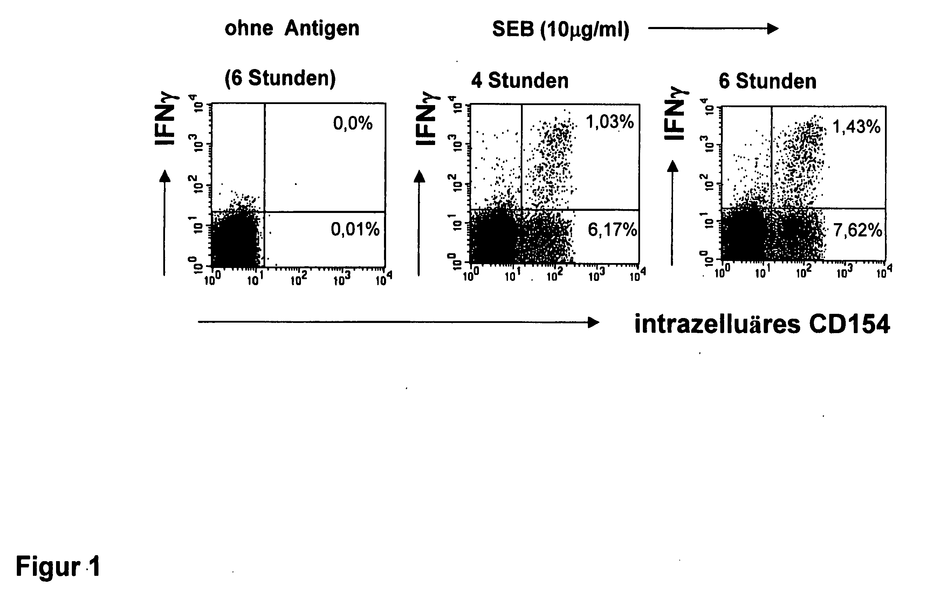

Die Kinetik der intrazellulären CD154 Expression in Antigen-reaktiven Th-Zellen nach *in vitro* Stimulation von humanen Vollblut mit dem Superantigen Staphylocokkus Enterotoxin B (SEB) wurde bestimmt. Jeweils 1ml Vollblut eines normalen gesunden Spenders wurde für die angegebenen Zeiten mit oder ohne SEB (1µg/ml) bei 37°C kultiviert. Für die letzten 2 Stunden der Kultur wurde der Sekretionsinhibitor BrefA zugegeben. Es sind auf CD4⁺ Th-Zellen begrenzte Analysen gezeigt. In Fig. 1 ist die Kinetik der intrazellulären CD154 Expression gezeigt.

### Beispiel 2

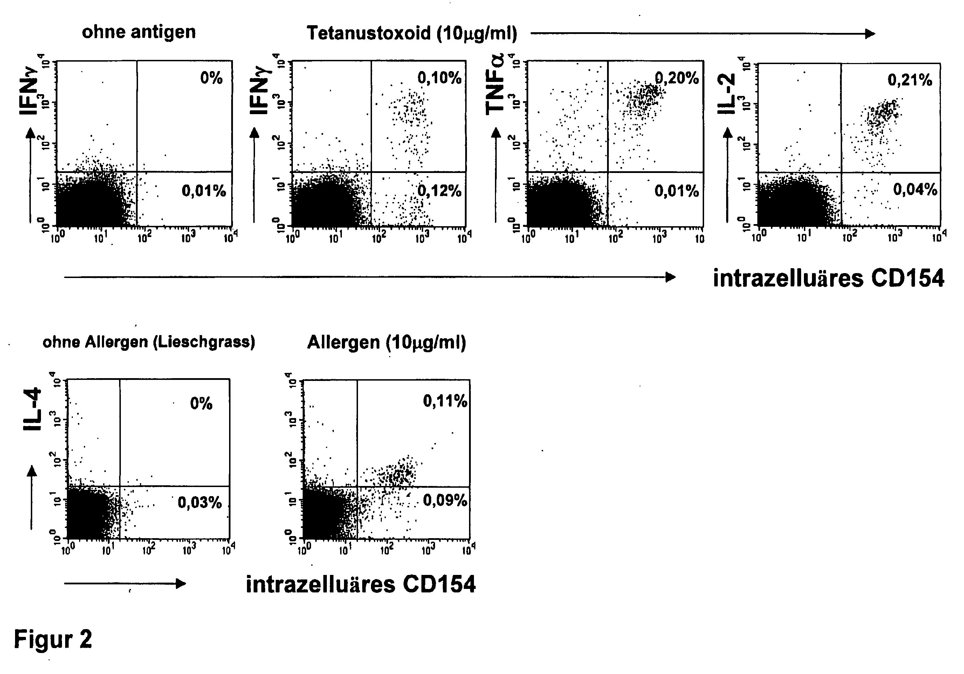

Fig. 2 zeigt den Nachweis Tetanus-Toxoid-(TT)-spezischer Th-Zellen und Allergen-spezischer Th-Zellen in humanem Vollblut unabhängig von der Art der reaktiv produzierten Zytokine nach *in vitro* Stimulation mit TT und Allergen. Jeweils 1ml Vollblut gesunder Spender wurde mit oder ohne die angegebenen Antigene (TT: 10µg/ml / Allergen: 20µg/ml) für 6 Stunden bei bei 37°C kultiviert. Für die letzten 4 Stunden wurde der Sekretionsinhibitor BrefA zugegeben. Es sind auf CD4⁺ Th-Zellen begrenzte Analysen gezeigt.

### Beispiel 3

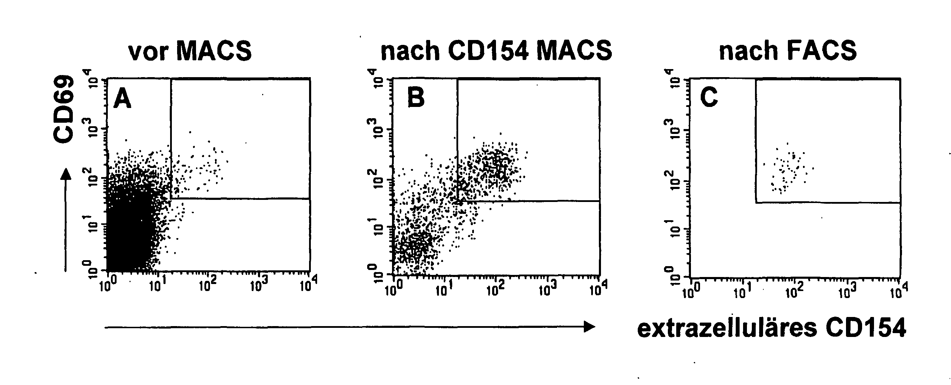

Die Isolierung von lebenden Tetanus-Toxoid-(TT)-spezifischen Th-Zellen anhand der reaktiven CD154 Expression aus humanen peripheren mononukleären Zellen nach in vitro Stimulation mit TT ist in Fig. 3 dargestellt. Aus ca. 20ml Volllblut wurden periphere mononukleäre Zellen gewonnen. Diese wurden für 6 Stunden mit 10µg/ml TT in Gegenwart eines monoklonalen anti-CD40 Antikörpers bei 37°C kultiviert. Alle Proben wurden mit Antikörpern gegen CD4-FITC, CD69-APC und CD154-PE markiert. Zum Auschluss von toten Zellen wurden Propidiumjodid verwendet. In 3A sind Analysen der CD154 Expression im Verhältnis zur Expression des Aktivierungsmarkers CD69 vor Isolierung gezeigt. Nach einer magnetischen Anreicherung mit PE-spezifische MicroBeads (3B) wurden lebende CD4⁺, CD154⁺ Th-Zellen weiter mit dem FACS aufgereinigt (Figure 3C). In den Figuren 3A und 3B sind die Analysen auf CD4⁺ Th-Zellen begrenzt.

## Patentansprüche

1. Verwendung von CD154 und eines CD40/CD154 Systeminhibitors, der die Interaktion wischen CD40 und CD154 blockiert oder inhibiert, zum in vitro Nachweis und/oder zur Isolierung von Antigen-spezifischen T-Lymphozyten.

2. Verwendung nach Anspruch 1,
**dadurch gekennzeichnet, dass**
als T-Lymphozyten CD4⁺ und/oder CD8⁺ T-Lymphozyten nachgewiesen und/oder isoliert werden.

3. Verwendung nach Anspruch 1 oder 2,
**dadurch gekennzeichnet, dass**
inflammatorische, antiinflammatorische, regulatorische und/oder suppressive T-Lymphozyten nachgewiesen und/oder isoliert werden.

4. Verfahren zum Nachweis und/oder Isolierung von Antigen-spezifischen T-Lymphozyten in einer Suspension nach Aktivierung mit einem Antigen, wobei die Suspension mit einem CD40/CD154 Systeminhibitor, der die Interaktion zwischen CD40 und CD154 blockiert oder inhibiert, in Kontakt gebracht wird, CD154 intra- oder extrazellulär bestimmt und die CD154 aufweisenden Zellen detektiert und/oder separiert werden.

5. Verfahren nach Anspruch 4,
**dadurch gekennzeichnet, dass**
als T-Lymphozyten CD4⁺ und/oder CD8⁺ T-Lymphozyten nachgewiesen und/oder isoliert werden.

6. Verfahren nach einem der Ansprüche 4 oder 5,
**dadurch gekennzeichnet, dass**
als CD40/CD154 Systeminhibitor ein anti-CD40-Antikörper, ein anti-CD154-Antikörper, ein Sekretionsinhibitor und/oder Endozytoseinhibitor verwendet werden.

7. Verfahren nach einem der Ansprüche 4 bis 6,
**dadurch gekennzeichnet, dass**
als Sekretionsinhibitor und/oder Endozytoseinhibitor Brefeldin A und/oder Monsensin verwendet werden.

8. Verfahren nach einem der Ansprüche 4 bis 7,
**dadurch gekennzeichnet, dass**
CD154 intrazellulär in fixierten Zellen detektiert wird.

9. Verfahren nach einem der Ansprüche 4 bis 8,
**dadurch gekennzeichnet, dass**
CD154 extrazellulär auf vitalen Zellen detektiert wird.

10. Verfahren nach einem der Ansprüche 4 bis 9,
**dadurch gekennzeichnet, dass**
die T-Lymphozyten isoliert und/oder separiert werden, die kein definiertes Zytokinmuster aufweisen.

## Claims

1. Use of CD154 and of a CD40/CD154 system inhibitor which blocks or inhibits the interaction between CD40 and CD154 for in vitro detection and/or isolation of antigen-specific T lymphocytes.

2. The use according to claim 1,
**characterized in that**
the T lymphocytes being detected and/or isolated are CD4⁺ and/or CD8⁺ T lymphocytes.

3. The use according to claim 1 or 2,
**characterized in that**
inflammatory, anti-inflammatory, regulatory and/or suppressive T lymphocytes are detected and/or isolated.

4. A method for the detection and/or isolation of antigen-specific T lymphocytes in a suspension following activation with an antigen, in which method the suspension is contacted with a CD40/CD154 system inhibitor which blocks or inhibits the interaction between CD40 and CD154, intra- or extracellular determination of CD154 is effected, and the cells having CD154 are detected and/or separated.

5. The method according to claim 4,
**characterized in that**
the T lymphocytes being detected and/or isolated are CD4⁺ and/or CD8⁺ T lymphocytes.

6. The method according to any of claims 4 or 5,
**characterized in that**
an anti-CD40 antibody, an anti-CD154 antibody, a secretion inhibitor and/or endocytosis inhibitor are used as CD40/CD154 system inhibitor.

7. The method according to any of claims 4 to 6,
**characterized in that**
brefeldin A and/or monsensin are used as secretion inhibitor and/or endocytosis inhibitor.

8. The method according to any of claims 4 to 7,
**characterized in that**
detection of CD154 is intracellular in fixed cells.

9. The method according to any of claims 4 to 8,
**characterized in that**
detection of CD154 is extracellular on vital cells.

10. The method according to any of claims 4 to 9,
**characterized in that**
T lymphocytes lacking a defined cytokine pattern are isolated and/or separated.

## Revendications

1. Utilisation de CD154 et d'un inhibiteur de système CD40/CD154, qui bloque ou inhibe l'interaction entre le CD40 et le CD154, pour détecter et/ou isoler in vitro des lymphocytes T spécifiques à un antigène.

2. Utilisation selon la revendication 1,
**caractérisée en ce**
**qu'**on détecte et/ou on isole, en tant que lymphocytes T, des lymphocytes T CD4⁺ et/ou CD8⁺.

3. Utilisation selon la revendication 1 ou 2,
**caractérisée en ce**
**qu'**on détecte et/ou on isole des lymphocytes T inflammatoires, anti-inflammatoires, régulateurs et/ou suppresseurs.

4. Méthode de détection et/ou d'isolation de lymphocytes T spécifiques à un antigène dans une suspension après activation avec un antigène, moyennant quoi on met en contact la suspension avec un inhibiteur de système CD40/CD154, qui bloque ou inhibe l'interaction entre le CD40 et le CD154, on détermine le CD154 de façon intra- ou extracellulaire et on détecte et/ou on sépare les cellules comportant du CD154.

5. Méthode selon la revendication 4,
**caractérisée en ce**
**qu'**on détecte et/ou isole, à titre de lymphocytes T, des lymphocytes T CD4⁺ et/ou CD8⁺.

6. Méthode selon une des revendications 4 ou 5,
**caractérisée en ce**
**qu'**on utilise, à titre d'inhibiteur de système CD40/CD154, un anticorps anti-CD40, un anticorps anti-CD154, un inhibiteur de sécrétion et/ou un inhibiteur d'endocytose.

7. Méthode selon l'une des revendications 4 à 6,
**caractérisée en ce**
**qu'**en tant qu'inhibiteur de sécrétion et/ou inhibiteur d'endocytose, on utilise du Brefeldin A et/ou du Monsensin.

8. Méthode selon l'une des revendications 4 à 7,
**caractérisée en ce**
**qu'**on détecte le CD154 de manière intracellulaire dans des cellules immobilisées.

9. Méthode selon l'une des revendications 4 à 8,
**caractérisée en ce**
**qu'**on détecte le CD154 de manière extracellulaire sur des cellules vitales.

10. Méthode selon l'une des revendications 4 à 9,
**caractérisée en ce**
**qu'**on isole et/ou on sépare les lymphocytes T, qui ne comportent aucun modèle de cytokine défini.
